# EUROPEAN PATENT APPLICATION

(11) **EP 4 722 340 A1**
(43) Date of publication of application: **08.04.2026**
(21) Application number: 24814693.8
(22) Date of filing: 08.04.2024
(51) Int. Cl.: C12N 5/02, B01J 19/06

(54) **METHOD FOR OBTAINING A CRYSTALLISED FOETAL BOVINE SERUM WITH AGMATINE**

(30) Priority: 01.06.2023 MX 2023006528
(71) Applicant: Moedano Lara, Raúl Francisco, Hidalgo, 42186 (MX)
(72) Inventor: Moedano Lara, Raúl Francisco, Hidalgo, 42186 (MX)
(74) Representative: Preusche und Partner Patent- und Rechtsanwälte mbB
(86) International application number: PCT/IB2024/053443
(87) International publication number: WO 2024/246625

(57) **Abstract**

The invention relates to a method for obtaining crystallised foetal bovine serum by adding agmatine that, at a specific concentration, acts to increase cell production and increase protein expression per cell. The method further relates to the specific form of reconstitution to liquid indicated in order to use same, without losing the original properties, allowing storage without an ultra-cold chain and reducing the risks of contamination, thereby allowing precise dosing of the amount to be used.

## Description

### FIELD OF THE INVENTION

The present invention relates to the field of biotechnology, specifically to a crystallization and reconstitution process for a supplement intended for use in cell culture media.

### OBJECT OF THE INVENTION

The object of the invention is a process for obtaining crystallized bovine fetal serum by incorporating agmatine, which was selected after testing various polyamines and identifying the one that most effectively enhances cellular production and increases protein expression per cell. The process achieves an optimal crystallization efficiency of 98% compared to liquid fetal serum, as well as an appropriate method of reconstitution into liquid form for specific use without loss of the original properties. This allows storage without the need for an ultra-cold chain and reduces contamination risks by enabling precise dosage according to the required volume of use.

### BACKGROUND OF THE INVENTION

For the cultivation of plant and animal cells, there is a constant need to employ protein-based sera that provide essential growth nutrients required for effective cellular development and for the production of the desired proteins or viruses. In order to achieve efficient development of biological products such as viruses or recombinant proteins, it is crucial to attain optimal cell density and to enhance intracellular protein expression, thereby maximizing the overall yield of the target product.

Generally, conventional formulations of bovine fetal serum require a cold chain for transportation and storage. The crystallized bovine fetal serum described in the present invention constitutes an innovative product presented in the form of fine crystals. This formulation can be conveniently adjusted to reduce undesirable proportions of albumin, transferrin, mycoplasma, and bovine spongiform encephalopathy, resulting in a safer product-particularly suitable for use in the manufacture of pharmaceuticals or vaccines intended for human application.

At present, several alternatives have been explored to provide effective culture conditions that significantly minimize contamination risks associated with thawing and extracting aliquots for laboratory use. The present invention arises from the need to offer a process that overcomes these and other technical limitations inherent in the bovine fetal sera currently available on the market.

The lot-to-lot variability associated with fluctuations in the concentrations of serum components and their biological activity can ultimately lead to experimental inconsistency and limit reproducibility among laboratories-particularly in the cultivation, expansion, and differentiation of primary cells-representing a significant cost factor in cell culture processes. Owing to the questionable reproducibility of models that rely on bovine fetal serum, its use for regulatory purposes remains under debate. For this reason, serum-supplemented media should be avoided whenever possible, and chemically defined media are preferred (van der Valk et al., 2018).

Ideally, a sufficient amount of serum should be available for the entire duration of a study. When switching to a new batch, researchers must ensure that no other reagents have been changed and that an adequate quantity of the previous serum is retained for verification purposes, in order to determine whether any unexpected results may be attributable to the batch change. It is, for example, easy to mistakenly conclude that an issue lies within a DNA transfection protocol when, in reality, a new serum batch has affected cell division rates. Researchers should also record the information provided by serum suppliers, including batch numbers (Baker, M., 2016).

To minimize serum contamination, some researchers are opting for gamma irradiation. Several common contaminants, including Mycoplasma, are sensitive even to low levels of radiation. However, this method requires a delicate balance, as irradiation may also damage growth proteins and bioactive molecules essential for cell viability. Cell culture consultant Raymond Nims, from RMC Pharmaceutical Solutions in Longmont, Colorado, advises that anyone planning to work with gamma-irradiated serum should first verify that the cells perform as expected, and bear in mind that even contaminant-free serum cannot prevent infections originating from other sources (Baker, M., 2016).

The use of serum-free media reduces animal suffering and may lead to more reproducible in vitro methods, some of which are being developed to replace or minimize animal testing, thereby contributing to the principles of replacement, reduction, and refinement of animal experimentation. Consequently, safety, scientific, and ethical considerations serve as major incentives for developing and implementing serum-free media (van der Valk et al., 2018).

In the present invention, we have successfully eliminated the need for a cold chain during the handling of the final product, allowing manipulation and dosage without risk of contamination and significantly reducing user costs by enabling storage at ambient temperature. We developed a crystallized bovine fetal serum obtained through classification, mixing, and treatment of various bovine fetal blood sources, yielding a serum that, upon crystallization and subsequent dissolution, reverts to its liquid form. When recrystallized, it is recovered in its solid state, which, once incorporated into a culture medium, provides the desired levels of cellular efficiency and productivity.

The innovation lies in the product's transformation process, given that the only dominant form available on the market is the conventional liquid bovine fetal serum. However, through a proprietary crystallization process, we have obtained this novel presentation, which constitutes a genuine technological advancement and a distinctive innovation in the field.

The crystallized bovine fetal serum disclosed in the present invention is a product presented in solid form, composed of fine crystals that preserve the same physicochemical and biological properties as the conventional liquid serum, without the need for freezing, and with greater efficiency and yield during handling. Among the advantages conferred by this product are: elimination of the cold chain, extended shelf life, capability for storage at ambient temperatures not exceeding 30 °C, elimination of refrigeration requirements, and the possibility of contamination-free dosing without compromising product integrity. Collectively, these characteristics render the product more efficient throughout distribution and commercialization processes.

The characterization of the crystallized bovine fetal serum underlying this invention is defined by the process through which the required quantities are prepared according to a dissolution reference table, enabling the preparation of suitable culture media. By simply reconstituting the crystals in a solution of pyrogen-free water, the bovine fetal serum can be regenerated with identical properties to those of the conventional liquid form.

Naturally, bovine fetal serum is conventionally supplied in liquid form; however, it is a highly sensitive and delicate product requiring careful handling, as it must be stored at a minimum temperature of -20 °C and can only be used once after thawing, due to the loss of its functional properties during the thawing process. Furthermore, commercial formulations of bovine fetal serum currently available on the market require a continuous cold chain for transportation and storage. The bovine fetal serum obtained through our crystallization process constitutes an innovative product presented as fine crystals, designed to reduce undesirable components such as albumin, transferrin, Mycoplasma, and the bovine spongiform encephalopathy virus, resulting in a safer product, particularly suitable for use in the manufacture of pharmaceuticals or vaccines intended for human application. Our invention enables uniform cellular growth and an increased yield of the desired products-particularly proteins. The distinctive feature of the present invention is the incorporation of agmatine, a short-chain polyamine, during the crystallization process of the bovine serum, which acts synergistically to enhance cell growth, specific cellular productivity, and final cell density.

Selected patents retrieved from patent databases under International Classification A61 - Human Necessities reference the state of the art within subclass A61K35/16. In particular, patent US2009124011A1, titled Serum Production System, discloses bovine serum compositions possessing controlled serum characteristics and methods for producing such compositions from the whole blood of the offspring of a female bovine mammal. This patent addresses the control of bovine fetal serum properties through specific production conditions; however, it does not disclose or suggest any crystallization process.

Within the sections A61D1/00, A61D1/08, A61K35/16, B01D17/00, B01D35/00, and B01D57/00, patent CN101112334A, titled Technology for Producing Bovine Fetal Blood Serum, relates to bovine fetal serum and, more specifically, to a process for producing such serum. The invention includes collection, separation, filtration, and subsequent freezing for storage. However, this patent does not disclose any crystallization process such as the one described in the present invention. None of the cited documents mention a method for crystallizing bovine fetal serum with agmatine, particularly under the specific parameters and conditions detailed in this application.

Interestingly, we have found that bovine fetal serum crystallized through our proprietary process is more favourable for both protein expression and cell growth rate when compared to conventional natural bovine fetal sera, which are generally considered standardized products with respect to their production processes.

### DESCRIPTION OF THE INVENTION

Our invention is described in detail, specifying its characteristics, function, and operational principles, with reference to the stages that comprise it and to the accompanying figures. Likewise, the distinctions between the present invention and the prior art are clearly identified.

### EXAMPLES

### Example 1.

Several analyses were conducted to determine the various parameters that the reference bovine fetal serum must meet, such as total protein and albumin content, in order to match or improve these characteristics in a different presentation - namely, the crystallized form.

| PARAMETERS | REFERENCE VALUES |
|---|---|
| TOTAL PROTEINS | 3.4-4.5 grams per deciliter |
| SERUM ALBUMIN | 1.2-2.5 grams per deciliter |
| GLOBULIN | 2.1-3.6 grams per deciliter |
| ALBUMIN-GLOBULIN RATIO | 1.1-1.4 |

The following results were obtained:

| BLOOD SAMPLE | PROTEIN | ALBUMIN | GLOBULIN |
|---|---|---|---|
| 1C | 3.30 grams per deciliter | 1.20 grams per deciliter | 1.10 grams per deciliter |
| 3C+1A | 3.50 grams per deciliter | 1.00 grams per deciliter | 1.50 grams per deciliter |
| B + ½ R | 3.60 grams per deciliter | 1.90 grams per deciliter | 1.70 grams per deciliter |
| B + 1R | 3.20 grams per deciliter | 1.60 grams per deciliter | 1.60 grams per deciliter |
| B + ½ R | 3.00 grams per deciliter | 1.10 grams per deciliter | 1.40 grams per deciliter |
| BFS (REF) | 3.0-4.5 grams per deciliter | 1.60-3.40 grams per deciliter | 1.70 grams per deciliter |

Once these batches of bovine fetal blood were identified, they were mixed to match the physicochemical characteristics of bovine fetal serum. The mixtures were defibrinated and centrifuged at 8,000 rpm for 30 minutes at 4 °C using a Beckman Coulter Avanti JxN-26 centrifuge. Subsequently, they were ultrafiltrated under nitrogen pressure employing Pall Acropack 0.10 µm double filters, and the resulting filtrates were transferred into sterile PETG containers.

Based on the tests conducted, the most significant batches yielding the most satisfactory results are listed below:

| BATCH | MIXTURE | |
|---|---|---|
| BATCH | BFS 3 MONTHS OF GESTATION | 400 ml |
| DQ-98/20-02-18 | BFS 7 MONTHS OF GESTATION | 400 ml |
| | BFS 11 MONTHS OF GESTATION | 400 ml |
| | SOL. TOTAL | 900 ml |

A mixture exhibiting characteristics comparable to the reference bovine fetal serum was obtained. These analyses were verified through testing conducted by a certified laboratory, LABORATORIOS LEI, where parameters such as total protein, albumin, immunoglobulins, endotoxins, osmolarity, hemoglobin, and hydrogen potential (pH) were confirmed. The corresponding laboratory results are attached as Annex "A".

From the second batch, prepared with an effective mixture, the following results were obtained:

| BATCH | MIXTURE | | REFERENCE IMAGE |
|---|---|---|---|
| BATCH | BFS MIXTURE | 800 ml | 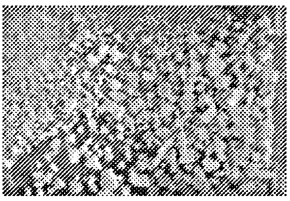 |
| DQ-145/18-04-19 | POLYAMINE | 100 ml | |
| | ETHANOL SOLUTION AT 40% | 100 ml | |
| | TOTAL SOLUTION: | 1000 ml | |

This batch was submitted to the Mexican Social Security Institute (IMSS) - Siglo XXI, specifically to the Immunology Department, where the same analyses were performed, this time including cell growth assays using HUVEC, RAJI, and HTP-1 cells. The corresponding laboratory results are attached as Annex "B".

| BATCH | MIXTURE | | REFERENCE IMAGE |
|---|---|---|---|
| BATCH | BFS | 500 ml | N/A |
| DQ-201-A/12-07-2019 | POLYAMINE | 100 ml | |
| ANALYSES: AUGUST 2019 | ETHANOL SOLUTION AT 40% | 80 ml | |
| | TOTAL SOLUTION | 760 ml | |
| BATCH | BFS 600 ml | | 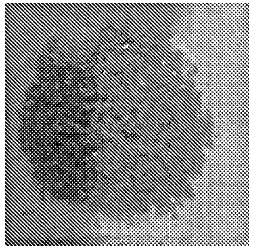 |
| DQ-201-B/16-10-2019 | POLYAMINE | 100 ml | |
| ANALYSIS: NOVEMBER 2019 | ETHANOL SOLUTION AT 40% | 80 ML | |
| | TOTAL SOLUTION | 780 ml | |

For the subsequent batch, the following mixture was prepared:
The sample was once again submitted to the Mexican Social Security Institute (IMSS) - Siglo XXI, specifically to the Immunology Department, where both physicochemical and cell growth tests were continued. Likewise, the sample was delivered to the National Polytechnic Institute (Instituto Politécnico Nacional), within the Biotechnology Department, where a cell culture assay using HeLa cells was performed. The corresponding laboratory results are attached as Annex "A." For the following batch, the mixture was prepared as follows:

| BATCH | MIXTURE | | REFERENCE IMAGE |
|---|---|---|---|
| BATCH | BFS | 600 ml | 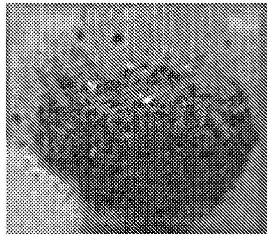 |
| DQ-297/20-01-2020 | POLYAMINE | 80 ml | |
| | ETHANOL SOLUTION AT 40% | 100 ml | |
| | TOTAL SOLUTION | 780 ml | |

| | | | |
|---|---|---|---|
| BFS: Bovine Fetal Serum. ES: Refers to a 40% Ethanol Solution. | | | |

Example 2. Work began on improving the physical properties of the product, such as consistency and color. To this end, lyophilization was considered and carried out at the Autonomous University of the State of Hidalgo (UAEH). However, the results were not satisfactory in terms of appearance or physicochemical properties, as they did not achieve the same characteristics as conventional bovine fetal serum.

Example 3. Another batch that yielded significant results is described below:

| BATCH | MIXTURE | REFERENCE IMAGE |
|---|---|---|
| BATCH | MIXTURE: BFS BATCH 297 + POLYAMINE + ES + Δ (CONTROLLED) | 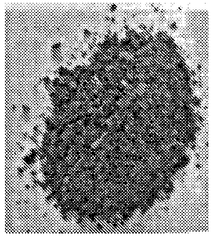 |
| DQ-326/14-11-2020 | | |
| | EFFICIENCY: 100 mL = 6.85 g | |

Several chemical compounds were subsequently employed to remove water and obtain the resulting solid, while testing various polyamines and polyamides until the desired results were achieved. A series of trial-and-error experiments were conducted to determine the specific organic reagent that produced the optimal outcome.

Example 4. For the subsequent batch, the following mixture was prepared:

| BATCH | MIXTURE | REFERENCE IMAGE |
|---|---|---|
| BATCH DQ-401/23-08-2021 | MIXTURE: BFS BATCH 297 + POLYAMINE + ES + Δ EFFICIENCY: 100 mL = 9.03 g | 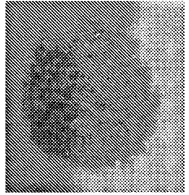 |

Work was again carried out under the same conditions as Batch DQ-297/14-12-2020, but in a larger volume. The same results as previously obtained were reproduced; however, this time the product was purified through the addition of ethyl alcohol, with the excess removed using a water bath, followed by drying at 30 °C and ultraviolet irradiation for 30-45 minutes to achieve sanitization.

Example 5. For product application, the required quantities are determined according to a dilution reference table for the preparation of the necessary culture media. By simply reconstituting the crystals in a solution of pyrogen-free water, the bovine fetal serum can be regenerated with the same properties as the conventional liquid form.

| DILUTION TABLE | | | |
|---|---|---|---|
| GRAMS OF CRYSTALLIZED BFS | MILLILITERS OF DILUENT | % FINAL SOLUTION FOR USE | EQUIVALENT IN MILLILITERS OF LIQUID BFS |
| 0.242 g | 50 ml | 10% | 5.0 ml |
| 0.485 g | 100 ml | 10% | 10 ml |
| 1.2125 g | 250 ml | 10% | 25 ml |
| 2.425 g | 500 ml | 10% | 50 ml |
| 4.85 g | 1000 ml | 10% | 100 ml |
| | | | |

| NOMENCLATURE | | | |
|---|---|---|---|
| ml | | Milliliters | |
| g | | Grams | |
| BFS | | Bovine Fetal Serum | |
| % | | Percentage | |

The above table was prepared based on 48.50 grams per liter of crystallized bovine fetal serum, from which the corresponding dilutions are derived. The efficiency of our process has ensured that the crystallized bovine fetal serum batches obtained contain consistent protein concentrations.

Once the bovine fetal serum has been crystallized, the specified quantities must be supplied in the diluent solution as indicated in the dilution table above, in order to achieve the target concentration in the corresponding culture medium, typically resulting in a 10% solution. The same table also provides the equivalent volume of liquid bovine fetal serum corresponding to the final reconstituted product.

FOR THE OBTAINMENT OF CRYSTALLIZED BOVINE FETAL SERUM, THE FOLLOWING PREFERRED EMBODIMENT OF THE INVENTION HAS BEEN SELECTED:
The process began with the exploration of various polyamides, among which certain aliphatic types were identified in the literature as being easily polymerizable, particularly under pH variations. Consequently, the focus shifted toward the study of polyamines, as the literature indicated that these compounds are more compatible with proteins-especially those of low molecular weight and low melting point. This led to the preparation of mixtures incorporating aliphatic compounds, where multiple experimental trials were conducted by modifying parameters such as consistency, appearance, colour, quantity, handling characteristics, polyamine concentration, alcohol concentration, temperature, dehydration conditions, and negative or positive chemical-biological effects, ultimately optimizing the crystallization process.

When work began with polyamines, the compounds agmatine, spermine, and spermidine were selected as available candidates. Each was subjected to the same experimental variables applied in previous polyamine tests, including assessments of consistency, appearance, colour, quantity, handling characteristics, polyamine concentration, ethyl alcohol concentration, temperature, dehydration conditions, and chemical and biological (positive-negative) effects, as well as crystallization behaviour.

Among these, the results presented herein correspond to the formulations that demonstrated the most favourable outcomes, exhibiting positive chemical and biological characteristics.

In a clear and sufficiently detailed manner, the following section describes the steps of the preferred embodiment of our invention - Process for Obtaining Crystallized Bovine Fetal Serum - to enable its practical implementation.
1. The origin of the animal (female bovine) is identified.
2. It is verified that the animal complies with all health and sanitary requirements, including a complete record of diseases, vaccinations, and feeding history.
3. One or more female bovines are selected that meet the following criteria:
   a) Up to 7 months of gestation.
   b) Free from disease.
   c) Fully vaccinated.
   d) Nutritionally healthy, maintained on a pasture-based diet.
4. The animal is humanely desensitized using a CACHETERO CASH device to ensure slaughter without cruelty.
5. The slaughter is performed by vertical exsanguination (neck incision).
6. The fetal drainage process is carried out to ensure complete removal of fluids.
7. The bovine foetus is then extracted vertically from the maternal cavity.
8. Fetal blood is collected vertically under strictly hygienic conditions into polypropylene bags to prevent contamination and preserve integrity for subsequent processing.
9. The collected blood is examined to ensure the absence of any external contaminants. A visual and manual inspection is performed to verify that the extraction process was carried out under appropriate sanitary conditions. Additionally, the sample is evaluated to determine whether coagulation has begun, as well as to assess key blood characteristics, including colour and density.
10. The blood is allowed to rest for five hours to enable coagulation, while being kept on ice to prevent microbial growth.
11. The blood is subjected to mechanical defibrination to break down any existing clots.
12. The defibrinated blood is then centrifuged to separate the serum at 8,000 rpm for 30 minutes at 4 °C using a Beckman Coulter Avanti JxN-26 centrifuge.
13. Decantation is performed.
14. Bovine fetal serum is thus obtained.
15. The serum is transferred into oval-shaped homogenization tanks with a capacity of 10 to 20 litres, where it is homogenized mechanically through circular, horizontal, vertical, and vibrational movements.
16. A homogeneous serum is obtained.
17. The serum is then transferred into stainless-steel cylindrical containers featuring an internal gradient, an external manometer to measure nitrogen pressure, a hermetically sealed lid, and a flexible conduit connecting the cylinder to a 10-20 litre receiving vessel. This system operates under nitrogen pressure to package the serum according to the desired dosage.
18. The serum is subjected to triple filtration using Pall Acropack double 0.1-micron filters to remove toxins and undesirable chemical compounds.
19. The product is packaged for distribution under hygienic conditions and in complete asepsis.
20. Once packaged, the serum is irradiated with ultraviolet light for a period of 30 to 45 minutes to ensure sterilization of the product.
21. The serum is then frozen at -21°C for storage.
22. The pure bovine fetal serum is thawed at an ambient temperature ranging from 8°C to 12°C.
23. Subsequently, 80 mL of a 30% agmatine solution in water is added, obtaining a pH of 6. The mixture comprises 600 mL of bovine fetal serum, 80 mL of 30% agmatine solution, and 100 mL of a 40% ethylic solution in water adjusted to pH 6.
24. The mixture is incubated for 2 hours at -5°C.
25. The compound is centrifuged at -6°C at a speed of 8,000 rpm to achieve separation of the solid matter.
26. If solids remain after centrifugation, the solid residues are discarded from the sample.
27. A 12.80% solution of ethyl alcohol at 40% concentration is added to the bovine fetal serum-agmatine compound to preserve the properties of the Bovine Fetal Serum without altering its chemical composition.
28. The mixture is subjected to centrifugation at 8,000 rpm at a maximum temperature of -5°C to separate the solid fraction.
29. Finally, the product is progressively exposed to a maximum temperature of 180°C for the time required to achieve complete water elimination and dehydration of the product, while maintaining its initial chemical properties.
30. The sample forms fine pink crystals, obtaining fine rose-colored crystals of approximately 1 micron.
31. Once the crystallized compound is obtained, it is packaged in sterile containers made of polyethylene terephthalate glycol (PETG) or glass.
32. It is irradiated with ultraviolet light for 30-45 minutes to ensure proper sanitization of the product.
33. Studies of the main parameters are carried out to verify compliance with the quality and asepsis requirements of the product.
34. For the use of the product, the required quantities are made available based on a dissolution table to prepare the necessary media; by simply regenerating the crystals in a solution of pyrogen-free water, bovine fetal serum can be obtained with the same properties as the known liquid form.

The quantities for the regeneration of the crystals and their optimal use are as follows: in 50 mL of pyrogen-free water solution, 0.242 g of crystallized bovine fetal serum; in 100 mL, 0.485 g of crystallized bovine fetal serum; in 250 mL, 1.2125 g of crystallized bovine fetal serum; in 500 mL, 2.425 g of crystallized bovine fetal serum; and in 1000 mL, 4.85 g of crystallized bovine fetal serum, according to the batch of the test performed.

## Claims

1. A process for obtaining a crystallized bovine fetal serum with agmatine, the process comprising the following steps:
1. identification of the origin of the female bovine;
2. verification of the health and sanitary requirements of the female bovine;
3. selection of the female bovine;
4. desensitization of the female bovine using the Cachetero Cash technique;
5. slaughter of the animal through vertical throat cutting;
6. draining of the bovine foetus;
7. extraction of the bovine foetus in a vertical position;
8. extraction of the fetal blood in a vertical position, collected under hygienic conditions in 25-liter polypropylene bags;
9. verification that the blood is free from any external contaminants;
10. resting of the blood;
11. mechanical defibrination of the blood to disrupt any type of coagulum;
12. separation of the serum by centrifugation;
13. decantation of the bovine fetal serum;
14. obtaining of the bovine fetal serum;
15. placement of the bovine fetal serum in homogenization tanks;
16. production of a homogeneous bovine fetal serum;
17. transfer of the bovine fetal serum into stainless steel containers;
18. filtration of the bovine fetal serum to remove toxins and undesirable chemical compounds;
19. packaging of the bovine fetal serum for distribution under hygienic conditions and in total asepsis;
20. sterilization of the bovine fetal serum;
21. freezing of the bovine fetal serum;
22. thawing of the pure bovine fetal serum;
23. preparation of a mixture composed of bovine fetal serum, agmatine, and water;
24. incubation of the mixture;
25. centrifugation of the mixture;
26. elimination of possible solids from the sample after centrifugation;
27. addition to the bovine fetal serum with agmatine of a 12.80% solution of ethyl alcohol at 40%, which helps to preserve the properties of the bovine fetal serum without affecting its chemical composition and to remove polyamine residues;
28. centrifugation for the separation of the solid matter;
29. drying of the mixture for dehydration;
30. obtaining of fine crystals;
31. packaging of the crystallized compound;
32. sanitization of the compound by ultraviolet light;
33. performance of quality control testing;
34. regeneration of the crystals.

2. The process for obtaining a crystallized bovine fetal serum with agmatine according to claim 1, **characterized in that** in step 3 the selection of the female bovine is based on a gestational age of up to 7 months.

3. The process for obtaining a crystallized compound of bovine fetal serum with agmatine according to claim 1, **characterized in that** in step 10 the resting period of the blood is 5 hours while kept on ice.

4. The process for obtaining a crystallized bovine fetal serum with agmatine according to claim 1, **characterized in that** in step 12 the separation of the serum is performed by centrifugation at 8,000 rpm for 30 minutes at 4°C using a Beckman Coulter centrifuge model Avanti JxN-26.

5. The process for obtaining a crystallized bovine fetal serum with agmatine according to claim 1, **characterized in that** in step 15 the tanks are made of stainless steel, have a capacity of 10 to 20 litres, and are oval-shaped, where homogenization is carried out by mechanical means, namely circular, horizontal, vertical and vibrational movements.

6. The process for obtaining a crystallized bovine fetal serum with agmatine according to claim 1, **characterized in that** in step 17 the serum is transferred to a stainless-steel cylinder connected to a nitrogen pressure system for packaging according to the desired dosage.

7. The process for obtaining a crystallized bovine fetal serum with agmatine according to claim 1, **characterized in that** in step 18 a triple filtration is performed with nitrogen pressure using Pall Acropack filters of 0.10 microns in double configuration.

8. The process for obtaining a crystallized bovine fetal serum with agmatine according to claim 1, **characterized in that** in step 20 the sterilization of the bovine fetal serum with ultraviolet light is carried out for 30-45 minutes.

9. The process for obtaining a crystallized bovine fetal serum with agmatine according to claim 1, **characterized in that** in step 21 the storage of the bovine fetal serum is at 21 degrees Celsius.

10. The process for obtaining a crystallized bovine fetal serum with agmatine according to claim 1, **characterized in that** in step 22 the thawing of the pure bovine fetal serum is carried out at an ambient temperature between 8 and 12 degrees Celsius.

11. The process for obtaining a crystallized bovine fetal serum with agmatine according to claim 1, **characterized in that** in step 23 the mixture is composed of 600 ml of bovine fetal serum, 80 ml of 30% agmatine, and 100 ml of 40% ethylic solution in water at pH 6.

12. The process for obtaining a crystallized bovine fetal serum with agmatine according to claim 1, **characterized in that** in step 24 the incubation of the mixture is for 2 hours at -5 degrees Celsius.

13. The process for obtaining a crystallized bovine fetal serum with agmatine according to claim 1, **characterized in that** in step 25 the centrifugation of the mixture at -6 degrees Celsius is performed at a speed of 8,000 rpm for the separation of solid matter.

14. The process for obtaining a crystallized bovine fetal serum with agmatine according to claim 1, **characterized in that** in step 27 ethyl alcohol is added to the compound of bovine fetal serum and agmatine, where the concentration of ethyl alcohol is 40%.

15. The process for obtaining a crystallized bovine fetal serum with agmatine according to claim 1, **characterized in that** in step 28 the centrifugation is performed at 8,000 rpm at a maximum temperature of -5°C for the separation of solid matter.

16. The process for obtaining a crystallized bovine fetal serum with agmatine according to claim 1, **characterized in that** in step 29 the drying temperature for dehydration of the compound progressively increases up to a maximum temperature of 180°C to prevent the loss of the initial chemical properties.

17. The process for obtaining a crystallized bovine fetal serum with agmatine according to claim 1, **characterized in that** in step 30 fine pink crystals are obtained with a size of 1 micron, not limited to 1 micron.

18. The process for obtaining a crystallized bovine fetal serum with agmatine according to claim 1, **characterized in that** in step 31 the packaging of the compound may be carried out in sterile containers made of polyethylene terephthalate glycol (PETG) or glass.

19. The process for obtaining a crystallized bovine fetal serum with agmatine according to claim 1, **characterized in that** in step 32 the sterilization of the compound is performed with ultraviolet light.

20. The process for obtaining a crystallized bovine fetal serum with agmatine according to claim 1, **characterized in that** in step 32 the sterilization time of the compound is 30-45 minutes.

21. The process for obtaining a crystallized bovine fetal serum with agmatine according to claim 1, **characterized in that** in step 34 the quantities for regeneration of the crystals and their use may be 50 ml of pyrogen-free water solution with 0.242 g of crystallized bovine fetal serum; 100 ml with 0.485 g of crystallized bovine fetal serum; 250 ml with 1.2125 g of crystallized bovine fetal serum; 500 ml with 2.425 g of crystallized bovine fetal serum; and 1000 ml with 4.85 g of crystallized bovine fetal serum.

22. A crystallized bovine fetal serum with agmatine obtained by the process according to claims 1 to 20.

23. The use of a crystallized bovine fetal serum with agmatine in cell cultures to increase cell production and enhance protein expression per cell.

24. The use of a crystallized bovine fetal serum with agmatine in cell cultures according to claim 1 for storage free from ultra-cold chain requirements.

25. The use of a crystallized bovine fetal serum with agmatine in cell cultures according to claim 1 using precise dosage according to claim 2 for the amount of use thereby reducing contamination risks.
